# EUROPEAN PATENT APPLICATION

(11) **EP 3 669 642 A1**
(43) Date of publication of application: **24.06.2020**
(21) Application number: 18382980.3
(22) Date of filing: 21.12.2018
(51) Int. Cl.: A01K 1/03, A01K 67/033

(54) **CAPSULE FOR EXPERIMENTS**

(71) Applicant: Asociación Centro de Investigación Cooperativa en Biociencias - CIC bioGUNE, 48160 Derio, Vizcaya (ES); Fundación Tekniker, 20600 Eibar (Guipúzkoa) (ES)
(72) Inventor: ANGUITA CASTILLO, Juan, 48160 Derio - Vizcaya (ES); MARTÍN RUIZ, Itziar, 48160 Derio - Vizcaya (ES); ALONSO EXTREMO, Jesús, 20600 Eibar - Guipúzcoa (ES); MENDIBIL BLASCO, Xabier, 20600 Eibar - Guipúzcoa (ES)
(74) Representative: ABG Intellectual Property Law, S.L.

(57) **Abstract**

The present invention is directed to a device for containing and placing ticks or other small sized animals on laboratory animals in order to perform controlled feeding experiments and a method of use thereof; in particular the present invention proposes a capsule (10) that is compact in size and easily accessible to permit feeding of small sized animals such as ticks on laboratory and larger animals.

## Description

### TECHNICAL FIELD OF THE INVENTION

The present invention is directed to a device for containing and placing ticks or other small sized arthropod animals on laboratory animals in order to perform controlled feeding experiments and a method of use thereof; in particular the present invention proposes a capsule that is compact in size and easily accessible to permit feeding of small sized animals such as ticks on laboratory and larger animals.

### BACKGROUND OF THE INVENTION

Arthropods constitute the largest animal phylum and are involved in nearly every kind of parasitic relationship, either as parasites themselves or as hosts/vectors for other micro-organisms (including viruses, bacteria, protozoa and helminths). They are generally ectoparasitic on, or in, the skin of vertebrate hosts. Many species are haematophagous (blood sucking) while others are histophagous (tissue-feeders) and bite or burrow in dermal tissues causing trauma, inflammation and hypersensitivity reactions. Infestations are transmitted from host-to-host either by direct contact or by free-living larvae, nymphs or adults actively seeking hosts.

The main arthropod assemblages include crustaceans (crabs, lobsters, crayfish, shrimp), arachnids (spiders, scorpions, ticks, mites) and insects (beetles, bugs, earwigs, ants, bees, termites, butterflies, moths, crickets, roaches, fleas, flies, mosquitoes, lice). Most parasitic arthropods belong to 2 main classes: the 6-legged insects (e.g. fleas, flies and lice), and the 8-legged arachnids (e.g. ticks and mites). Parasitic arthropods are able to transmit a great variety of microorganisms, many of them capable of inducing disease in humans and domestic animals. Diseases transmitted by ticks include Lyme borreliosis, anaplasmosis, babesiosis or tick-borne encephalitis. Some of these diseases are cause of serious concerns and even death. Therefore, research aiming to understand how ticks (and other arthropods) feed, the process of microbial transmission and the test of potential treatments that can prevent these processes and therefore, infection, are of great importance. The need to study arthropod feeding is compounded by the lack of viable vaccines against most of the microorganisms transmitted by these animals. Thus, studies using arthropods are also being carried out in order to find suitable and effective vaccine candidates to prevent arthropod feeding and the transmission of infectious agents.

The study of tick feeding is usually performed in rodents, including mice, rabbits or guinea pigs. These studies are also performed in large animals, including cows and sheep, which are natural hosts of these arthropods. These experiments require the placement of the ticks on the skin of the animal under controlled conditions and allow them to feed for different periods of time until completion, which can take up to a week. The management of tick populations is complicated by their active nature and their tendency to migrate to secluded areas in the surface of the skin of the animal, so that their follow-up and control is more complicated. Currently, investigators use home-made devices that contain the ticks and apply them to certain skin areas, from where the ticks can be easily managed.

### SUMMARY OF THE INVENTION

The present invention provides an alternative solution for the aforementioned problems, by a capsule according to claim 1 and a method of use thereof according to claim 13. In dependent claims, preferred embodiments of the invention are defined.

In a first inventive aspect, the invention provides a *capsule suitable for conducting experiments with animals, comprising*
*a capsule body with a hollow configuration, defining an outer side and an inner side, or cavity, the capsule body comprising at least a wall, and at least a first opening and a second opening communicating the cavity with the outer side of the capsule,*
*wherein the capsule further comprises at least first closing means and second closing means, adapted to close the at least first opening and the second opening, respectively, and*
*wherein the capsule comprises a flange with a flat configuration, and arranged along a perimeter of the second opening and projecting from the wall of the capsule body.*

Throughout all this document it will be understood that the experiments which can be conducted with the capsule are preferably controlled feeding experiments of small sized arthropods on laboratory animals or larger animals. Other possible uses of the capsule are feeding experiments of arthropods on devices which simulate the skin of a laboratory animal, or other arthropod feeding devices.

Although preferred, the invention is not only applicable for use with arthropods. Throughout this document, for simplicity, small sized animals, small sized arthropods, arthropods, arthropod animals, ticks or mites should be understood as alternative names for the animals which are placed inside the capsule and are fed under controlled conditions; accordingly, these arthropod animals must be smaller than the capsule. Further, it should be understood that the experiments are conducted by bringing the arthropods to contact with a surface with arthropod food. In one embodiment, the arthropod food is animal blood, and the surface is the skin of a laboratory animal or a larger animal; examples of these laboratory or larger animals are mice, rabbits, guinea pigs, sheep or cows.

Advantageously, several small sized animals can be contained inside the cavity of the capsule and conveniently placed on a surface in order to perform controlled feeding experiments; particularly, several arthropods can be brought to contact with the skin of a laboratory animal for feeding purposes by means of a capsule which is compact in size, reliable, safe and easily accessible to the investigators. In a particular embodiment, *the capsule body has a cylindrical or prismatic configuration, wherein the first opening and the second opening are positioned at opposite bases of the cylindrical or prismatic capsule body.*

Advantageously, a cylindrical or prismatic configuration provides with a simple yet robust configuration for a capsule, which in addition can be manufactured by usual manufacturing processes, such as molding or extrusion.

In a particular embodiment, *the flange has a flat annular configuration and projects radially from the wall of the capsule body.*

In the particular embodiment, when the body has a cylindrical or prismatic configuration, the flat annular flange is comprised in the plane defined by the second base of the cylinder or prismatic base.

Advantageously, the flat annular configuration of the flange provides with a wide rim which can be used as a support base for the capsule, both during storage and in use attached to a surface. If this surface is the skin of an animal, the flat annular flange allows a better contact between the capsule and the skin, and provides with a possible holding point for any holding means, such as adhesive tape or bandages.

In a particular embodiment, *the capsule body has a tubular configuration.* In another particular embodiment, the cavity is cylindrical, and the outer side of the body has a prismatic configuration with a different cross-section.

Tubular configuration should be understood as a cylinder with a through cylindrical hole. Advantageously, the cylindrical configuration of the cavity avoids slots or recesses where small sized animals can hid.

Throughout this document it should be understood that cylindrical configuration includes not only the shape of a straight cylinder, but also slight variations thereof, such as tapered cylinder shape, cone shape, hourglass shape or barrel shape.

In a particular embodiment, *the first closing means, or the second closing means, or both are detachable lids.*

Advantageously, the lids allow for a simple and reliable closing means for the cavity; the lids are reusable and can be easily placed and removed in the openings as many times as desired.

In a particular embodiment, *the first closing means or second closing means, or both comprise a flexible junction attaching the closing means with the capsule.*

Advantageously, a junction which can be bent without damage to the material keeps the lids attached to the comparatively bigger capsule, and thus avoids their loss during manipulation. In a further improvement, the flexible junction is made of the same material as the lids and the body of the capsule. In another embodiment, the body of the capsule, one of or both lids and the flexible junction are manufactured in one and the same part.

In a particular embodiment, *the second closing means is a detachable film.*

Advantageously, the detachable film provides with a simple, reliable and cost efficient closing means, whose production can be easily automatized. As a further advantage, the film provides with a very thin closing means, resulting in a more compact capsule which is easier to store.

In a particular embodiment, *the flange comprises a layer of adhesion means adapted to adhere the capsule to a surface, preferably to the skin of a mammal.*

Advantageously, the layer of adhesion means provides with built-in means for attaching the capsule to a surface. If this surface is the skin of an animal, it is particularly advantageous to provide with some reliable attaching means, for example an adhesive surface which keeps the capsule adhered to the skin while the animal moves or interacts with the environment.

In a particular embodiment, *the flange comprises a flexible porous layer adapted to conform to the irregularities of a surface.*

Advantageously, the flexible porous layer is a layer of material interposed between a surface, for example the skin of an animal, and the flange of the capsule which allows for a better contact between an irregular surface, such as the skin of an animal, and the capsule. In a particular embodiment, the layer of flexible porous material is positioned between a layer of adhesion means and the flange.

In a particular embodiment, *the inner diameter of the body is between 10,0 mm and 95,0 mm, and*/*or the outer diameter of the body is between 15,0 mm and 100,0 mm, and*/*or the height of the body is between 12,0 mm and 35,0 mm.*

Advantageously, a plurality of capsule sizes enable the use of the capsule in a range of applications, such as attaching the capsule to the skin of laboratory or larger animals of different sizes, like for example mice, rabbits, guinea pigs, sheep or cows.

In a particular embodiment, *the thickness of the flange is between 0,2 mm and 1,0 mm, and*/*or wherein preferably the flange projects in a radial direction from the body between 2,0 mm and 15,0 mm.*

Advantageously, a wide flange provides a broad seat or support of the capsule, thus enabling a better attachment to the surface.

In a particular embodiment, *part* of *or all the elements of the capsule are made of a transparent and flexible material, preferably the elements of the capsule are made of an elastomer.*

Advantageously, a transparent capsule shows the content of the cavity to the external observer or investigator for monitoring purposes. A flexible material, that is a material which is elastically deformable without compromising the integrity of the object, improves the comfort of the animal on to which the capsule is attached.

In a particular embodiment, the flexible porous layer preferably is made of ethylene-vinyl acetate (EVA) foam.

Advantageously, the EVA foam adapts to the irregularities of the skin and enables a durable, safe and reliable adhesion between the capsule and the skin of the animal, regardless of the movements of the animal.

In a particular embodiment, part of or all the elements of the capsule are made of an elastomer, preferably polyurethane or polydimethylsiloxane (PDMS).

Advantageously, the polyurethane or polydimethylsiloxane fulfil the requirements of a transparent, flexible and resistant material.

In a second inventive aspect, the invention provides a *method for using a capsule according to the first inventive aspect in order to conduct an experiment with animals, comprising the steps of*
*a) providing a capsule according to the first inventive aspect, in which the at least first and second openings are closed;*
*b) opening the first opening of the capsule and placing in the cavity a number of small sized animals, preferably arthropods;*
*c) closing the first opening with the first closing means;*
*d) removing the second opening means of the capsule; and*
*e) attaching the capsule to a surface, preferably the skin of a mammal, supporting the flange on said surface, in such a way that the small sized animals can contact the surface, and in such a way that they are not allowed to escape from the cavity.*

Advantageously, the method for using a capsule allows an investigator to manipulate, feed and observe small sized animals, particularly ticks in a controlled environment, specifically the cavity of the capsule. The investigator can do so in a clean, safe and convenient way.

All the features described in this specification (including the claims, description and drawings) and/or all the steps of the described method can be combined in any combination, with the exception of combinations of such mutually exclusive features and/or steps.

### DESCRIPTION OF THE DRAWINGS

These and other characteristics and advantages of the invention will become clearly understood in view of the detailed description of the invention which becomes apparent from a preferred embodiment of the invention, given just as an example and not being limited thereto, with reference to the drawings.
- Figures 1a-1b: These figures show a perspective view of a capsule and a cross section view of a capsule, with the closing means extracted.
- Figures 2a-2b: These figures show a perspective view of a capsule and a cross section view of a capsule, with the closing means in place.
- Figure 3a, 3b, 3c: These figures show a perspective view of a capsule and two cross section views of a capsule, with detachable film closing means.

### DETAILED DESCRIPTION OF THE INVENTION

The main purpose of the capsule (10) is containing small sized animals, such as ticks or other arthropods, within a closed, controlled environment, in such a way that they can be put into contact with the skin of an animal. While in contact, the ticks are allowed to feed directly from the skin. To this aim, rodents are preferred as subjects of experimentation, but it is also possible to feed the ticks on the skin of a goat, sheep or cow. In a particular case, the living tissue can be replaced by an artificial surface interposed between the ticks and the food.

Ticks are relatively small and prone to hid in small cavities, therefore, the inner side of the capsule (10), that is the cavity (1), is preferably a smooth continuous surface without corners, slots or recesses, such as a cylinder. In a simple embodiment, the external cross section of the body (3) of the capsule (10) is circular, while in other embodiments (not shown in the figures) the cross section of the body (3) is polygonal or square, with or without rounded edges.

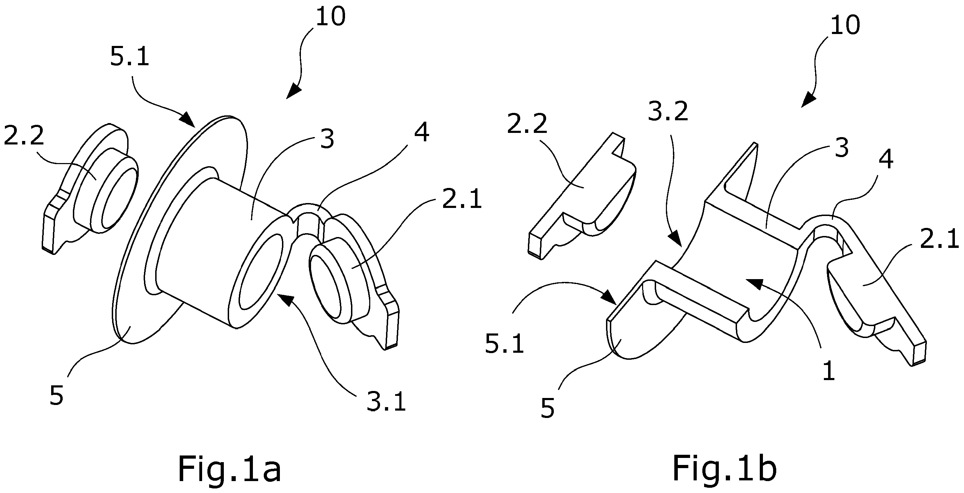

The embodiment shown on Figures 1a and 1b comprises a cylindrical body (3) with a cylindrical cavity (1); the openings (3.1, 3.2) of the body (3) are made at both bases of the cylinder, giving rise to a tubular configuration of the capsule (10), and are closed by two detachable lids (2.1, 2.2), which on Figures 1a and 1b are depicted detached from the capsule (10). These lids (2.1, 2.2), or plugs, comprise a small protrusion or knob of a smaller diameter than the outer diameter of the body (3), and are introduced in the respective openings (3.1, 3.2) in order to close the cavity (1). The lids (2.1, 2.2) are kept in place by interference fit with the body (3); this is achieved by designing the protrusion of the lids (2.1, 2.2) with a slightly bigger diameter than the inner diameter of the body (3). Also, in the embodiment shown on Figures 1a and 1b, the lids (2.1, 2.2) comprise a small lug to improve the grip while pulling said lids (2.1, 2.2) for opening the capsule (10).

In alternative embodiments (not shown in the figures), the lids (2.1, 2.2) comprise a rim configured to fit over the external side of the openings (3.1, 3.2) and the wall (3.3) of the body, in such a way that, when closed, the lids (2.1, 2.2) do not reduce the available volume of the cavity (1).

The body (3) can be made of a plurality of materials, providing that the structural integrity of the capsule (10) is achieved; however it is particularly advantageous the use of an elastomer, for example polyurethane or polydimethylsiloxane (PDMS). These materials are strong enough to produce the minimum structural strength, and at the same time provide with some flexibility, so that it can absorb minor elastic deformation. This flexibility enhances the comfort of the animal on which skin the capsule (10) is placed.

In the same embodiment shown on Figures 1a and 1b, the flange (5) is a flat, annular or ring shaped rim contained in the plane of the second base of the cylindrical body (3), substantially around the second opening (3.2). With the help of the flange (5), the capsule (10) can be conveniently attached to a surface, either the skin of an animal or an arthropod food vessel covered by a film. In this embodiment, the flange (5) is an integral part of the body (3). As can be observed on Figure 1a, there is a transition region between the body (3) and the flange (5); with this transition region the concentration of stresses in the connection area is reduced.

In one particular embodiment, the thickness of the flange (5) is between 0,2 mm and 1,0 mm, and the flange (5) projects in a radial direction from the body (3) between 2,0 mm and 15,0 mm.

Also a flexible junction (4) is configured as a small strip attached to both the body (3) and the lids (2.1, 2.2); preferably, the flexible junction (4) is made of the same material as the body (3), and prevents the loss of the lids during the manipulation of the capsule (10). The flexible junction (4) is depicted on all Figures 1a to 3c, both with open and closed lids (2.1, 2.2).

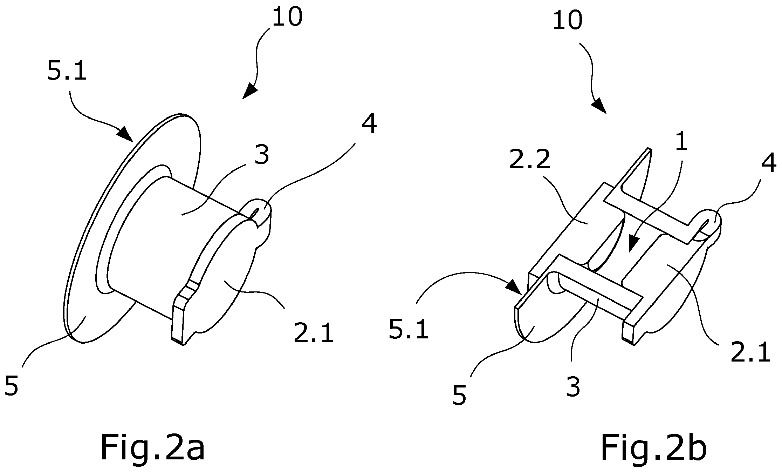

Figures 2a and 2b show the same embodiment as on Figures 1a and 1b, with the lids (2.1, 2.2) in a closed position. Comparing Figures 1b and 2b, it is possible to distinguish the dimensions of the cavity (1) and its smooth configuration, without corners.

The embodiment of Figures 1a to 2b also comprises a layer of adhesion means (5.1) which will be discussed further on.

In one embodiment, the inner diameter of the body (3) is between 10,0 mm and 95,0 mm, the outer diameter of the body (3) is between 15,0 mm and 100,0 mm, and the height of the body (3) is between 12,0 mm and 35,0 mm.

These dimension ranges are intended to fit laboratory animals of different sizes, for example mice, guinea pigs, rabbits, cows or sheep. In an indicative way, the approximate dimensions of the body (3) for each animal are as follows:

| **Animal** | **Inner diameter (mm)** | **Outer diameter (mm)** | **Height (mm)** |
|---|---|---|---|
| Mice | 10±5 | 15±7 | 12±6 |
| Guinea pigs | 25±12 | 30±15 | 20±10 |
| Rabbits | 35±17 | 40±20 | 30±15 |
| Cows or sheeps | 95±47 | 100±50 | 35±17 |

According to the previous table, a capsule (10) for feeding arthropods on a rabbit measures, in a specific embodiment, 35 mm of inner diameter, 40 mm of outer diameter and 30 mm of height.

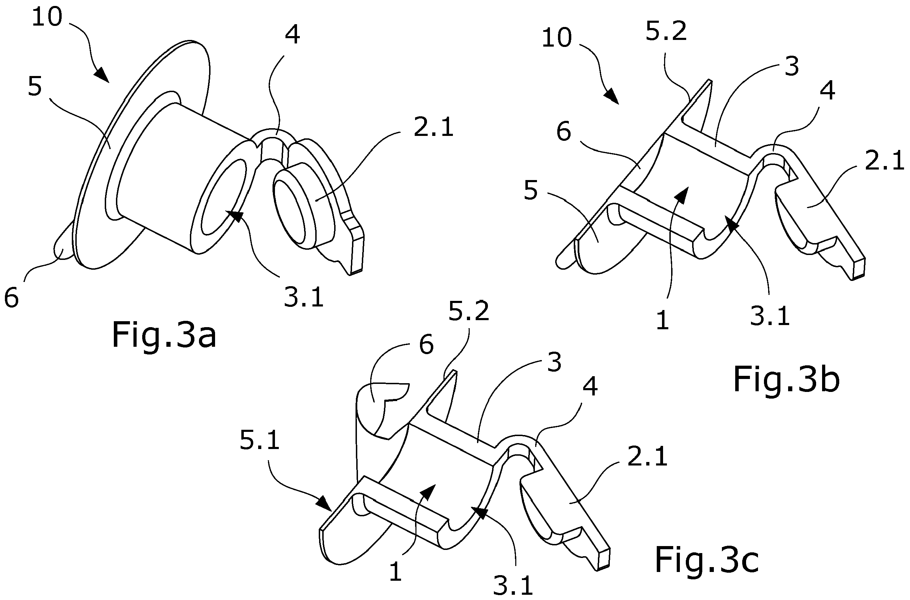

On Figures 3a, 3b, and 3c it is depicted another embodiment of the invention; this particular embodiment comprises a detachable film (6) in place of the second lid (2.2). This detachable film (6) is preferably attached during the production of the capsule (10) to the wide, outer surface of the flange (5) covering the second opening (3.2), and it is preferably removed at the moment of its use, once the ticks have been introduced in the cavity (1) and the first lid (2.1) has been closed.

This embodiment further comprises a layer of adhesion means (5.1); this layer of adhesion means (5.1) is intended to enhance the attachment of the capsule (10) with the surface, and in one particular embodiment is a double faced adhesive film with the same dimensions as the flange (5), with an opening corresponding to the second opening (3.2), which enables the communication between the cavity (1) and the surface of the skin.

As an additional improvement, this embodiment comprises a flexible porous layer (5.2) adapted to conform to the irregularities of a surface. This flexible porous layer (5.2) is preferably configured in the same way as the layer of adhesion means (5.1), that is, with the same dimensions as the flange (5) and an opening for access to the cavity (1), and it is advantageously positioned between the flange (5) and said adhesion means (5.1). In turn, the adhesion means (5.1) are covered with the detachable film (6) thus protecting the adhesion means (5.1) from soiling and closing the cavity (1). Consequently, the flexible porous layer (5.2) enables a reliable attachment of the capsule (10) in spite of the skin irregularities.

Both the layer of adhesion means (5.1) and the flexible porous layer (5.2) can be applied to the embodiment shown on Figures 1a to 2b.

Therefore, an investigator may use a capsule (10) as described in one or many of the previous embodiments for performing controlled feeding experiments of arthropods on laboratory animals according to the following method or procedure:
With all the openings (3.1, 3.2) closed, the first lid (2.1) is removed, and a small batch of small sized animals is carefully introduced in the cavity (1); the first lid (2.1) is then closed again, taking care not to allow the small sized animals to escape. Then, the second opening (3.2) is opened, either by extracting a lid (2.2) or by removing the film (6). Then, with the second opening (3.2) exposed to the environment, the capsule (10) is attached to the skin of an animal or another surface suitable for animal feeding. If the capsule (10) comprises a layer of adhesion means (5.1), it is simply pressed against the animal skin until the adhesion is achieved; in another case, additional means shall be provided to achieve the attachment, such as the use of adhesive tape or bandages.

## Claims

1. Capsule (10) suitable for conducting experiments with animals, comprising
a capsule body (3) with a hollow configuration, defining an outer side and an inner side, or cavity (1), the capsule body (3) comprising at least a wall (3.3), and at least a first opening (3.1) and a second opening (3.2) communicating the cavity (1) with the outer side of the capsule (10),
wherein the capsule (10) further comprises at least first closing means (2.1) and second closing means (2.2), adapted to close the at least first opening (3.1) and the second opening (3.2), respectively, and
wherein the capsule (10) comprises a flange (5) with a flat configuration, and arranged along a perimeter of the second opening (3.2) and projecting from the wall (3.3) of the capsule body (3).

2. Capsule (10) according to the previous claim, wherein the capsule body (3) has a cylindrical or prismatic configuration, wherein the first opening (3.1) and the second opening (3.2) are positioned at opposite bases of the cylindrical or prismatic capsule body (3).

3. Capsule (10) according to the previous claim, wherein the flange (5) has a flat annular configuration and projects radially from the wall (3.3) of the capsule body (3).

4. Capsule (10) according to any of the previous claims 2 or 3, wherein the capsule body (3) has a tubular configuration.

5. Capsule (10) according to any of the previous claims, wherein the first closing means (2.1), or the second closing means (2.2), or both are detachable lids.

6. Capsule (10) according to any of the previous claims, wherein the first closing means (2.1) or second closing means (2.2), or both comprise a flexible junction (4) attaching the closing means (2.1, 2.2) with the capsule (10).

7. Capsule (10) according to any of the previous claims, wherein the second closing means (2.2) is a detachable film (6).

8. Capsule (10) according any of the previous claims, wherein the flange (5) comprises a layer of adhesion means (5.1) adapted to adhere the capsule (10) to a surface, preferably to the skin of a mammal.

9. Capsule (10) according any of the previous claims, wherein the flange (5) comprises a flexible porous layer (5.2) adapted to conform to the irregularities of a surface.

10. Capsule (10) according any of the previous claims, wherein the inner diameter of the body (3) is between 10,0 mm and 95,0 mm, the outer diameter of the body (3) is between 15,0 mm and 100,0 mm, and the height of the body (3) is between 12,0 mm and 35,0 mm.

11. Capsule (10) according any of the previous claims, wherein the thickness of the flange (5) is between 0,2 mm and 1,0 mm, and wherein the flange (5) projects in a radial direction from the body (3) between 2,0 mm and 15,0 mm.

12. Capsule (10) according any of the previous claims, wherein part of or all the elements of the capsule (10) are made of a transparent and flexible material, preferably the elements of the capsule (10) are made of an elastomer.

13. Method for using a capsule (10) according to any of claims 1 to 12 in order to conduct an experiment with animals, comprising the steps of
a) providing a capsule (10) according to any of claims 1 to 12, in which the at least first and second openings (3.1, 3.2) are closed;
b) opening the first opening (3.1) of the capsule (10) and placing in the cavity (1) a number of small sized animals, preferably arthropods;
c) closing the first opening (3.1) with the first closing means (2.1);
d) removing the second opening means (2.2) of the capsule (10); and
e) attaching the capsule (10) to a surface, preferably the skin of a mammal, supporting the flange (5) on said surface, in such a way that the small sized animals can contact the surface, and in such a way that they are not allowed to escape from the cavity (1).
